# EUROPEAN PATENT SPECIFICATION

(11) **EP 3 515 312 B1**
(45) Date of publication and mention of the grant of the patent: **05.07.2023**
(21) Application number: 16788196.0
(22) Date of filing: 20.09.2016
(51) Int. Cl.: A61B 6/14, A61B 6/00, A61B 5/00, G16H 30/20, G16H 40/63, A61C 9/00, A61B 6/03

(54) **IMAGE ACQUISITION IN DARKROOM MODE**
BILDAUFNAHME IM DUNKELKAMMERMODUS
ACQUISITION D'IMAGE EN MODE CHAMBRE NOIRE

(43) Date of publication of application: 31.07.2019
(73) Proprietor: Trophy, 77435 Marne La Vallée Cedex 2 (FR)
(72) Inventor: FAURE, Michel, 77435 Marne la Vallée Cedex 2 (FR); DUMOUSSEAUX, Fréderic, 77435 Marne la Vallée Cedex 2 (FR)
(74) Representative: Santarelli
(86) International application number: PCT/IB2016/001449
(87) International publication number: WO 2018/055426

(56) References cited:
- EP-A1- 1 953 566
- EP-A1- 2 394 579
- EP-A1- 2 722 003
- EP-A1- 2 767 237
- US-A1- 2007 172 112
- US-A1- 2007 258 638
- US-A1- 2011 311 026

## Description

### FIELD OF THE INVENTION

The disclosure relates generally to methods and apparatus for diagnostic imaging and more particularly to methods and apparatus that provide darkroom-quality image viewing workspace with utilities for acquiring and displaying new image content.

### BACKGROUND OF THE INVENTION

High-contrast and high-detail image presentation can have considerable diagnostic value for medical and dental practitioners. Viewing of x-ray images of teeth or other anatomical structures at the highest available resolution enables the practitioner to more properly characterize the anatomical structures and to plan and execute treatment strategies with improved accuracy and confidence.

For the sake of illustration, EP 2 722 003 discloses devices for presentation of digital radiographic images that can be displayed on a display in a first mode and in a light box mode that simulates the appearance of a physical light box. EP 2 767 237 discloses systems and methods for inspecting data generated during a scan of an object, by generating a signal to display a cinematic sequence in a frame-by-frame manner. US 2011/0311026 discloses a mobile radiography apparatus having a display configured to provide an examination procedure for a patient, including a visual indication that at least one related prior image exists for the examination procedure.

A utility that is acknowledged to be of particular value for diagnostics and treatment is the use of a darkroom mode presentation that provides full-screen visibility of the anatomy of interest. Darkroom mode emulates the proven presentation methods preferred by practitioners, who previously viewed x-ray and other images of the anatomy under carefully controlled lighting conditions, using backlighting effects to help accentuate detail and to reduce or eliminate distraction from stray light and other visual noise that can otherwise obscure the presentation of teeth and other anatomy of interest. Generally, darkroom mode enables to select and display any image in a pure black environment with no graphic user interface other than a small toolbar reminding of the patient name whose images are being viewed and enabling to scroll through all already opened images. In darkroom mode, a selection of tools can be accessed such as through a right click on the image. In a darkroom mode, the displayed view of a tooth or other structure can show high levels of detail and contrast during evaluation and planning stages as well as at intervals during treatment itself. Darkroom mode imaging presentation displays the image over the full display screen or substantially over the full display screen.

One aspect of darkroom mode presentation relates to constraints imposed by the software that is conventionally used for this purpose. In an effort to reproduce the conventional backlit viewing conditions familiar to the film-based x-ray environment, darkroom presentation software eliminates unnecessary or distracting information fields from the displayed view. While this is helpful for analysis of individual images, there are some practical disadvantages to this arrangement. For example, the successive procedures in the workflow for a particular treatment sequence can require the practitioner to exit darkroom mode presentation in order to acquire interim images that show the progression of treatment. Thus, in the case of endodontics or orthodontics, for example, the practitioner views images that show the existing state of the treatment process, then exits the darkroom display, enters a separate image acquisition utility to obtain and upload the next image needed for evaluating progress in the treatment sequence, and then re-enters the darkroom mode. Thus, there is a need for an improved darkroom mode capability.

### SUMMARY OF THE INVENTION

It is an object of the present disclosure to advance the art of dental diagnostic imaging by addressing the need for a workflow sequence that supports darkroom viewing without the added complexity, keystrokes, and/or time needed for updating image content related to treatment progress.

Another object of this application is to address, in whole or in part, at least the foregoing and other deficiencies in the related art.

It is another object of this application to provide, in whole or in part, at least the advantages described herein.

Exemplary method and/or apparatus embodiments according to the present disclosure can provide darkroom mode imaging with tools and utilities for readily acquiring new image content as desired or needed, without the requirement to terminate or suspend darkroom imaging during this time and/or without the requirement that some other system, outside the darkroom imaging system, provide the image acquisition function. The newly acquired image content can be of any suitable type, depending on the capabilities of the dental imaging apparatus that is used for a specific application and available at a treatment site during darkroom mode imaging.

These objects are given only by way of illustrative example, and such objects may be exemplary of one or more embodiments of the invention. Other desirable objectives and advantages inherently achieved by the disclosed methods may occur or become apparent to those skilled in the art. The invention is defined by the appended claims.

According to an aspect of the present disclosure, there is provided a dental imaging apparatus that can include at least one image acquisition device that acquires an image of patient dentition; and a host processor that is in signal communication with a display and is energizable to generate a graphical user interface on the display, the graphical user interface (GUI) comprising a darkroom mode GUI configured to display only one selected diagnostic image of one or more diagnostic images of a selected patient, the darkroom mode GUI including a display area to display the selected displayed diagnostic image among the one or more diagnostic images of a selected patient, a first user control configured to exit the darkroom mode GUI upon selection, a second user control configured to alternate the selected displayed diagnostic image in the display area of the darkroom mode GUI among the one or more diagnostic images of a selected patient, and a third user control configured to prompt an operator of a dental image acquisition device to manually energize dental image acquisition by the dental image acquisition device, where the dental image acquisition device is in signal communication with the darkroom mode GUI, and where the third user control is selectable only when the dental image acquisition device is capable of immediate dental image acquisition of the selected patient.

### BRIEF DESCRIPTION OF THE DRAWINGS

The foregoing and other objects, features, and advantages of the invention will be apparent from the following more particular description of the embodiments of the invention, as illustrated in the accompanying drawings.

The elements of the drawings are not necessarily to scale relative to each other. Some exaggeration may be useful in order to emphasize basic structural relationships, operator interface capabilities, or principles of operation.
Figure 1 shows components of a dental imaging system for obtaining and displaying patient images of various types, including images acquired during different stages of a treatment session.
Figure 2 shows a darkroom mode display according to an embodiment of the present disclosure, for darkroom mode imaging of teeth.
Figure 3 shows darkroom mode display following an entered viewer instruction.
Figure 4 shows an additional overlay with command buttons for entry of additional viewer instructions related to the selected teeth.
Figure 5A shows an x-ray interface window activated and used by the viewer.
Figure 5B shows, in schematic form, an alternate example for display of an intraoral image.
Figure 6 shows a darkroom image display during one stage of endodontic treatment,
Figure 7 shows an example of a thumbnail image gallery for a darkroom imaging system.

### DESCRIPTION OF EXEMPLARY EMBODIMENTS

The following is a detailed description of exemplary embodiments, reference being made to the drawings in which the same reference numerals identify the same elements of structure in each of the several figures.

Where they are used in the context of the present disclosure, the terms "first", "second", and so on, do not necessarily denote any ordinal, sequential, or priority relation, but are simply used to more clearly distinguish one step, element, or set of elements from another, unless specified otherwise.

As used herein, the term "energizable" relates to a device or set of components that perform an indicated function upon receiving power and, optionally, upon receiving an enabling signal.

In the context of the present disclosure, the terms "viewer", "operator", and "user" are considered to be equivalent and refer to the viewing practitioner, technician, or other person who may operate an imaging system, camera, or scanner and may also view and manipulate the presentation of an image, such as a dental image, on a display monitor. An "operator instruction" or "viewer instruction" is obtained from explicit commands entered by the viewer, such as by clicking a button on the camera or scanner or by using a computer mouse or by touch screen or keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

In the context of the present disclosure, the term "imaging apparatus" relates to a device that is enabled to acquire any of a number of types of images, using an appropriate source of light radiation, including various types of reflectance imaging, structured light imaging, panoramic imaging, x-ray imaging, computed tomography (CT) imaging, cone beam computed tomography (CBCT) imaging, or other imaging type.

The term "subject" refers to the tooth or other portion of a patient that is imaged and displayed.

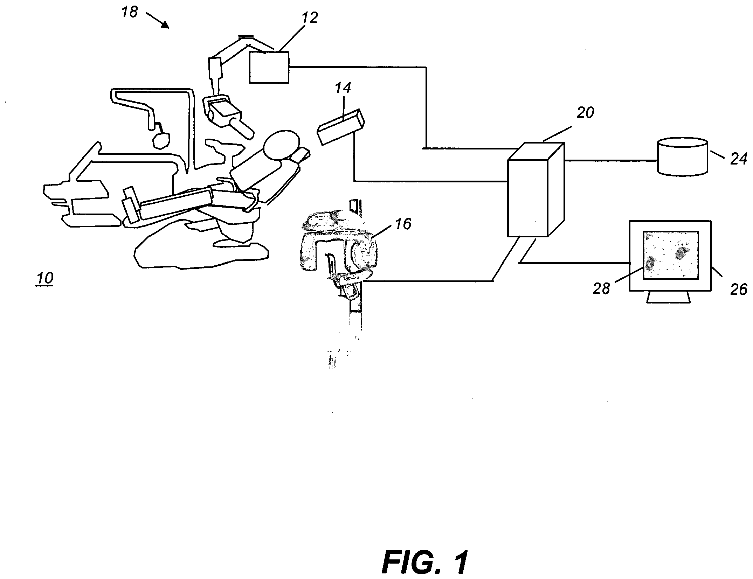

Figure 1 shows components of a dental imaging system 10 for obtaining and displaying patient images of various types using darkroom mode imaging or darkroom image presentation, including images of patient dentition acquired during different stages of a treatment session. Dental imaging system 10 includes at least one imaging apparatus 18 for image acquisition, which may be an x-ray imaging apparatus 12 using a digital detector or other film or computed radiography detector with an appropriate scanner, a digital camera 14 such as an intra-oral camera or scanner, such as a structured light imaging scanner, or a dental cone-beam computed tomography (CBCT) system 16 for generating volume images of tooth structure. Other types of imaging apparatus 18 could also be employed for obtaining images of teeth and supporting structures, gums, and related tissue, such as panoramic imaging apparatus, ultrasound imaging apparatus, depth resolved images or other imaging types coupled to dental imaging system 10. In addition, various types of diagnostic measurement instrumentation may also be provided for working with dental imaging system 10, as described in more detail subsequently.

Still referring to Figure 1, a host processor 20, such as a computer or other type of dedicated logic processor for obtaining, processing, and storing image data from the imaging apparatus 18 is also part of dental imaging system 10, along with one or more displays 26 for viewing image results. Host processor 20 provides the link to each of one or more image sources that can provide image content for display in darkroom mode. Host processor 20 is in signal communication with display 26 and is energizable to generate a graphical user interface on the display, the graphical user interface (GUI) including a darkroom mode GUI.

Each display 26 can have graphical user interface (GUI) 28 for entry of viewer instructions related to image display functions. GUI 28 can use standard entry devices including a touch screen or other instruction entry device, such as a keyboard, mouse or other pointer. Host processor 20 is in data communication with one or more image capture devices for image acquisition and, optionally, with any number of automated measurement devices (not shown in Figure 1). In addition, host processor 20 can also be in data communication with a database of patient records, stored internally or on a networked host or server, for example. The memory 24 can include or access the database of patient records, information, dental diagnostic images and/or dental practice management applications. A computer-accessible memory 24 is also provided, which may be a non-volatile memory storage device used for longer term storage, such as a device using magnetic, optical, or other data storage media. In addition, computer-accessible memory 24 can comprise a volatile electronic memory such as a random access memory (RAM) within or otherwise in data communication with host processor 20, that is used for shorter term data storage, such as memory used in conjunction with a display device for temporarily storing image content as a display buffer, or memory that is employed to store a computer program having instructions for controlling one or more computers to practice exemplary method and/or apparatus embodiments according to the present disclosure.

Exemplary darkroom method and/or apparatus embodiments according to the present disclosure can provide darkroom mode imaging with tools and utilities for readily acquiring new image content as desired or needed, without the requirement to terminate or suspend darkroom imaging during this time and/or without the requirement that some other system mode or utility, outside the darkroom imaging system, provide the image acquisition function. Certain exemplary darkroom method and/or apparatus embodiments can enable to select and display any 2D/3D image (e.g., DR/CR/Color/Full mouth series) in a pure black environment with no graphic user interface other than a small toolbar reminding of the patient name whose images are being viewed or acquired, and enabling to scroll through all already opened images or open new images or acquire new images. In darkroom mode, a selection of tools can be accessed such as through a right click on the image. Further, in darkroom mode, access to available system toolbars or a list of any toolbar can be provided.

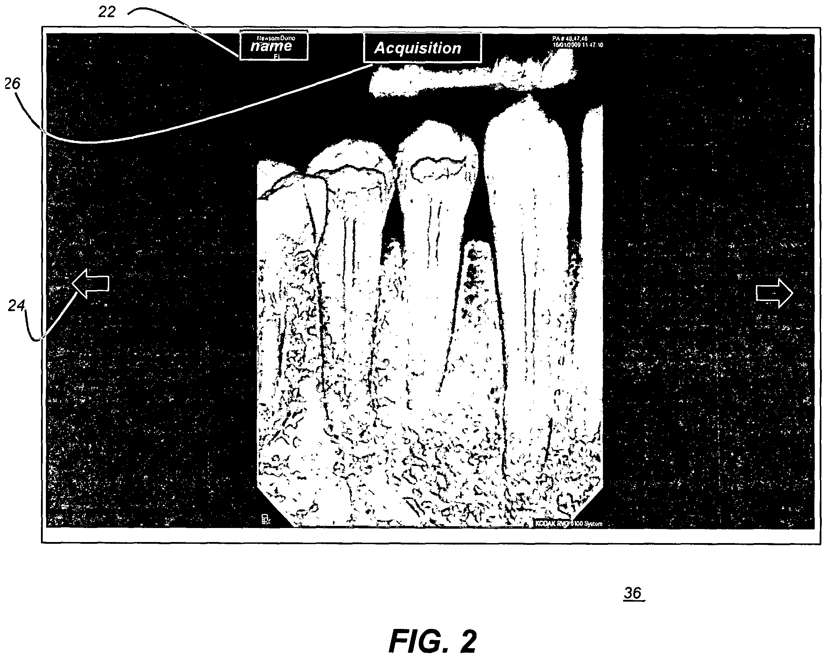

Figure 2 shows a darkroom mode display according to an exemplary embodiment of the present disclosure, for darkroom mode presentation of images of teeth. Emulating conventional backlit presentation used for x-ray viewing, darkroom mode display or GUI 20 in Figure 2 shows a full-screen view of the imaged patient dentition. In the absence of operator instructions, display 20 can utilize the full display screen 36; the darkroom image extends to the dimensional limits of the display in at least one dimension, without obstruction from selectable instruction entry icons. Darkroom mode display 20 can include patient name 22. Darkroom mode display 20 can include scroll selection(s) 24. Darkroom mode display 20 can include image acquisition selection 26 that can specify different types of image acquisition (when coupled imaging apparatus 18 are available).

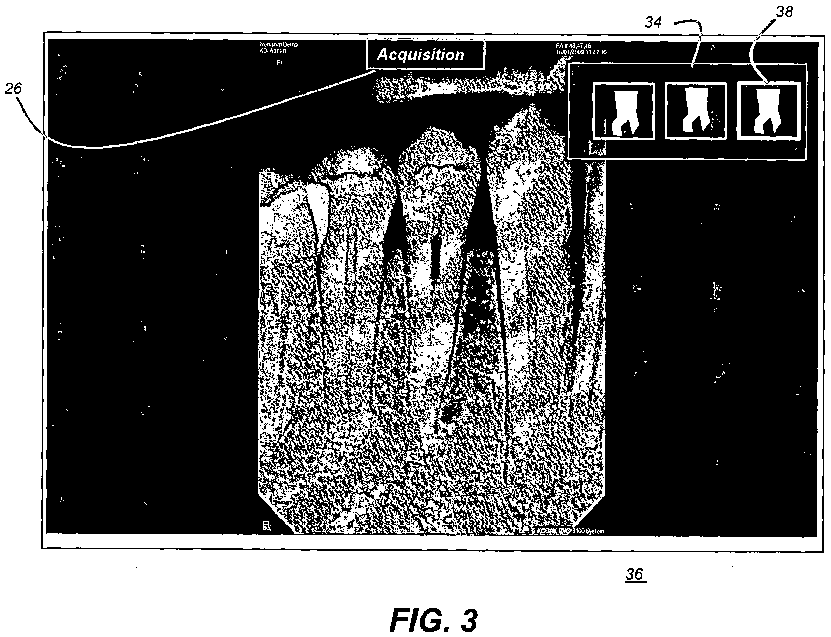

Figure 3 shows an exemplary darkroom mode display 20 following an entered viewer instruction, such as a swipe or tapping against a touch screen display 20 or an instruction from a mouse or a keyboard entry, specifying the image for darkroom mode display. In one embodiment, Figure 3 can be displayed by selecting and opening patient dental image(s) in a 2D image viewer and then selecting darkroom mode. An overlay (e.g., filmstrip) 34 shows a mapping with at least one thumbnail image 38, allowing selection of one of opened patient images of dentition. Overlay 34 can be partially transparent to allow some visibility to the background image underneath. The relative transparency of the overlay 34 can be adjusted by the viewer.

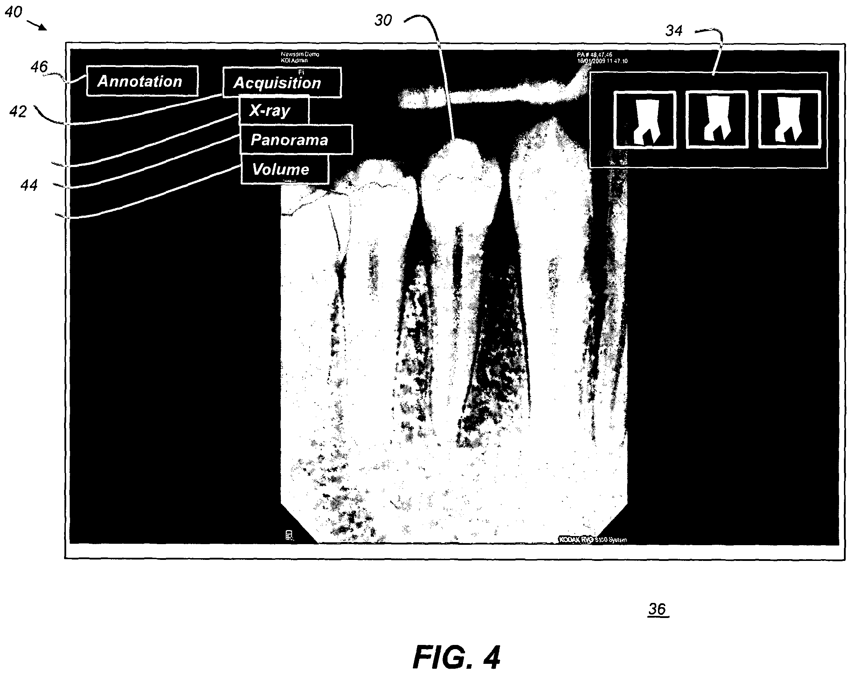

Figure 4 shows an additional overlay 40, that provides a toolbar menu with command buttons for image-related utilities including entry of additional viewer instructions related to the selected teeth. In some exemplary embodiments, an acquisition button 42 activates additional buttons 44 that can specify different types of image acquisition available with dental imaging system 10 (Figure 1), used for the selected teeth, for example. The particular buttons 44 that can be activated (when a corresponding dental imaging system is available) can show acquisition settings, equipment on/off and status, and other detailed information obtained from related components of the system. At a particular site, the detailed information that is available can depend on the equipment setup at the time and can vary based on factors such as patient position, on/off state of the imaging apparatus, and other parameters. It should be emphasized that buttons 44 do not exercise control over image acquisition; darkroom mode 20 is not intended as a "control panel" for initiating display acquisition itself. Instead, buttons 44 provide information about linked devices for image acquisition. This information can include status and properties data for any connected image acquisition device. Other toolbar menu selections can include drawing and measurements utilities, image viewer workspace utilities, export and print toolbar, a share toolbar or patient information toolbar, for example. Still other menu selections can generate histogram displays for the currently displayed image. Zoom and pan capabilities can also be available as toolbar menu selections. In one embodiment, control buttons or the like can allow selection of one or more teeth 30 from the displayed image. Then, embodiments of the application can be used to select the acquisition button 42 to show additional buttons 44 that can specify different types of image acquisition available with dental imaging system 10 for the one or more selected teeth 30, for example. As shown in Figure 4, an optional annotation button 46 allows viewer entry of text related to a particular image, tooth, or treatment. Any suitable annotation utility can be used for entering text for practitioner notes and observations.

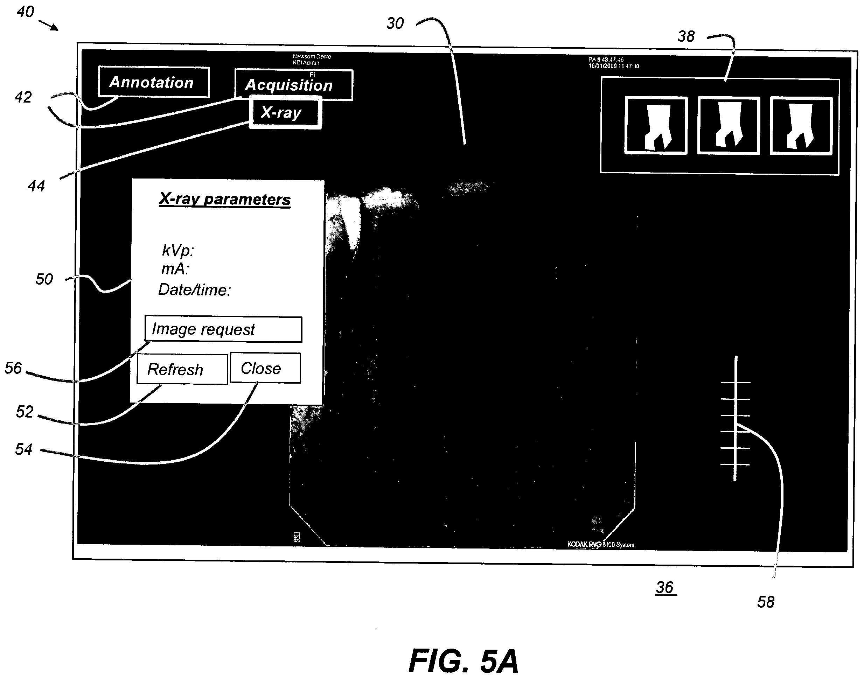

By way of example, referring to Figure 5A, touching the x-ray button 44 actuates an operator interface window 50. Here, the display lists different operational parameters of the x-ray imaging apparatus 12 (Figure 1) used in order to obtain the currently displayed x-ray image in darkroom mode.

Selecting control button 56 can prompt an operator of a dental image acquisition device 18 to manually energize dental image acquisition by the dental image acquisition device. Thus, for example, the operator of an x-ray of CBCT apparatus can be instructed to acquire one or more additional images, as desired or needed by the darkroom image viewer. Control button 56 may be activated only when the corresponding dental image acquisition apparatus is capable of immediate dental image acquisition of the selected patient. The transmitted requests can be sent without requiring the system to exit darkroom mode. Preferably, the newly acquired image can then be viewed still without leaving darkroom mode. Transmitted requests can also be entered by the operator of a dental image acquisition device. The control button 56 can be highlighted when an external request is entered, such as to prompt the viewer to enter and thus verify the command. Selection from the acquisition device is available only when the dental image acquisition device is capable of immediate dental image acquisition of the selected patient.

Pressing the Refresh button 52 obtains another image acquisition from the same device and updates the listed parameters. In one embodiment, window 50 can provide the operator actions to change the parameters from the default operational parameters used to obtain the currently displayed patient image in the darkroom mode. Pressing the Close button 54 can remove the operator interface window 50. In one exemplary embodiment, pressing the Close button 54 can be used to exit darkroom mode display for the selected acquisition device, such as to restore the display to a default acquisition device or to a previous image, for example. An optional measurement tool 58 can also be provided, such as with a scale for placement against the displayed view of one or more of the teeth or other anatomical structures for obtaining dimensional values of interest.

In addition to the x-ray example shown in Figure 5A, the imaging system can be linked with other types of extraoral image acquisition systems. For example, a volume image, such as an image reconstructed using a CBCT image scan can also be acquired and displayed, along with its associated parameters, using the appropriate control button 44. At the GUI, any of the obtained images can be acquired, processed, and displayed without exiting the darkroom mode, eliminating the need to terminate or suspend darkroom image presentation in order to acquire updated or alternate types of image data.

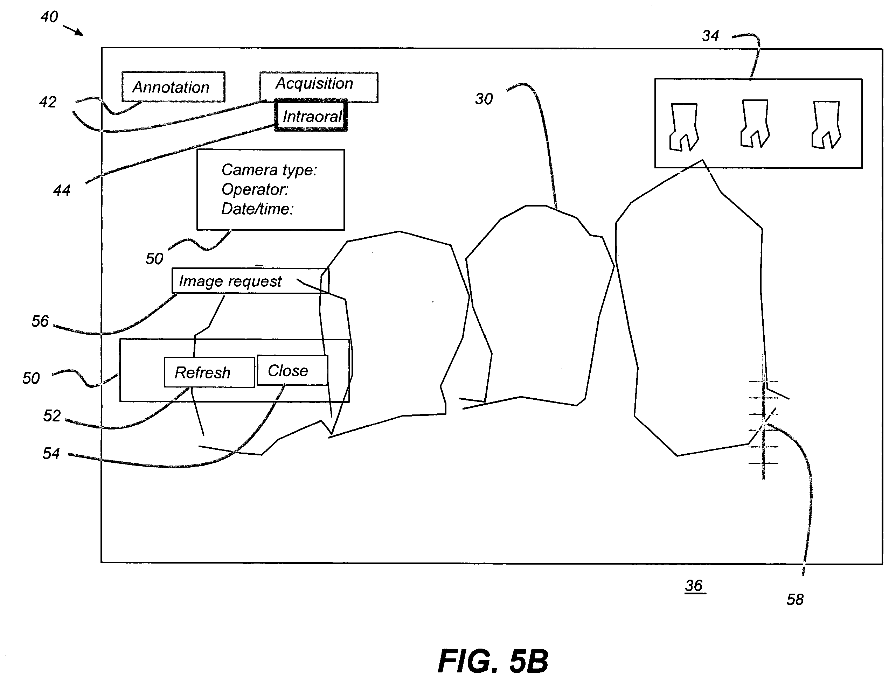

Figure 5B shows, in schematic form, an alternate example for display of an intraoral image, with control button 44 linking to an intraoral digital camera device for display of image content, such as color or monochromatic images, from an intraoral camera. Operator interface window 50 provides basic status and properties information from the intraoral camera. Control button 52 provides an instruction for refresh, enabling access to more recently obtained images. Control button 54 can provide a close command to remove window 50 and/or to terminate the link with the intraoral camera.

Color images can also be acquired where a color camera is provided. Linkage to structured light images for shape characterization and 3D mesh generation can also be activated and parameters displayed. A full mouth scan can be obtained, or a partial mouth scan, according to the teeth selected.

A succession of images can also be viewed, enabling the practitioner to view each tooth in succession as well as allowing the practitioner to view each individual tooth during a different step in treatment.

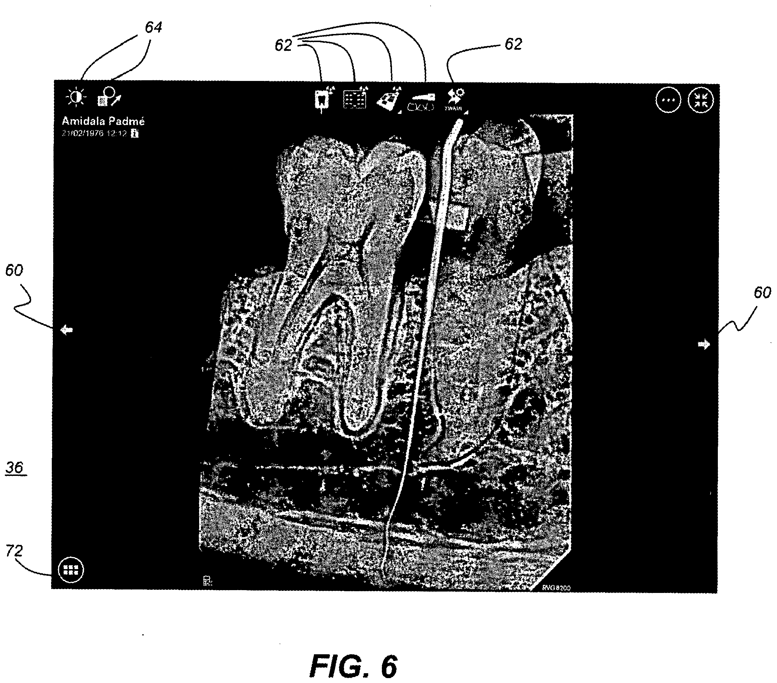

By way of example, Figure 6 shows a darkroom image display 20 during one stage of endodontic treatment. A set of control icons 64 enables adjustment of display characteristics such as brightness and contrast.

As the practitioner works with the affected tooth, the progress of the procedure can be repeatedly checked using exemplary darkroom method and/or apparatus embodiments described herein, allowing the practitioner to acquire successive images at appropriate intervals. Continuing with the description of Figure 6, forward and backward arrows 60 allow the practitioner to page through successive images from the treatment sequence or to page through successive images of teeth or alternate views of the same tooth. A set of image acquisition linkage buttons 62 displays along the header of the screen display. Each button 62 provides linkage and communication with image acquisition and display devices to provide suitable images for darkroom mode display. For example, buttons 62 can be used to acquire the following types of images:
(i) x -ray image;
(ii) full-mouth scan;
(iii) 3D volume image;
(iv) intraoral camera image, using operator assistance;
(v) camera or scanner image using a standard image interface mechanism such as the TWAIN Applications Programming Interface (API).

Buttons 62 can display with appropriate highlighting or enhanced brightness to show an active/de-activated mode depending on connection and power-up state of the corresponding image acquisition device.

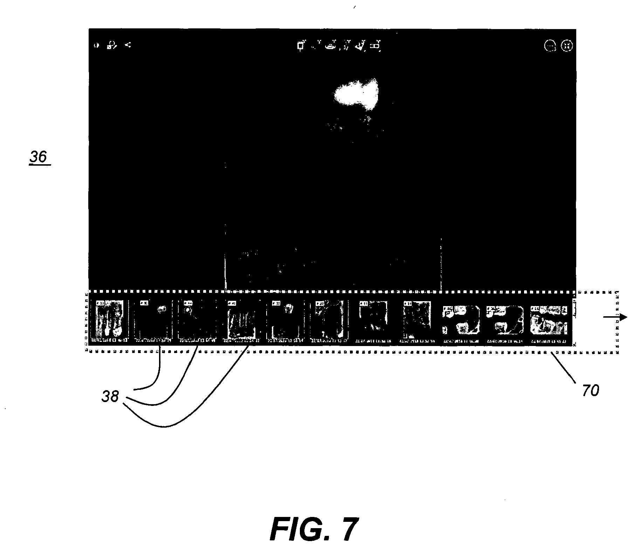

Other features can be added to enhance darkroom viewing capabilities with minimal impact on display screen surface area or "real-estate". Figure 7 shows an example of a thumbnail image gallery 70 that can be selectively displayed or removed from visibility. Image gallery 70 allows selection of an image, from its thumbnail representation, for full-size darkroom display. A control button 72 can be used to generate or remove the image gallery 70.

Consistent with exemplary embodiments herein, a computer program can use stored instructions that perform on image data that is accessed from an electronic memory. As can be appreciated by those skilled in the image processing arts, a computer program for operating the imaging system and probe and acquiring image data in exemplary embodiments of the application can be utilized by a suitable, general-purpose computer system operating as host processor 20 as described herein, such as a personal computer or workstation. However, many other types of computer systems can be used to execute the computer program of the present invention, including an arrangement of networked processors, for example. The computer program for performing exemplary method embodiments may be stored in a computer readable storage medium. This medium may include, for example; magnetic storage media such as a magnetic disk such as a hard drive or removable device or magnetic tape; optical storage media such as an optical disc, optical tape, or machine readable optical encoding; solid state electronic storage devices such as random access memory (RAM), or read only memory (ROM); or any other physical device or medium employed to store a computer program. Computer programs for performing exemplary method embodiments may also be stored on computer readable storage medium that is connected to the image processor by way of the internet or other network or communication medium. Those skilled in the art will further readily recognize that the equivalent of such a computer program product may also be constructed in hardware.

It should be noted that the term "memory", equivalent to "computer-accessible memory" in the context of the application, can refer to any type of temporary or more enduring data storage workspace used for storing and operating upon image data and accessible to a computer system, including a database, for example. The memory could be non-volatile, using, for example, a long-term storage medium such as magnetic or optical storage. Alternately, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that is directly associated with a display device and is periodically refreshed as needed in order to provide displayed data. This temporary storage buffer is also considered to be a type of memory, as the term is used in the application. Memory is also used as the data workspace for executing and storing intermediate and final results of calculations and other processing. Computer-accessible memory can be volatile, non-volatile, or a hybrid combination of volatile and non-volatile types.

It will be understood that computer program products of the application may make use of various image manipulation algorithms and processes that are well known. It will be further understood that computer program product exemplary embodiments of the application may embody algorithms and processes not specifically shown or described herein that are useful for implementation. Such algorithms and processes may include conventional utilities that are within the ordinary skill of the image processing arts. Additional aspects of such algorithms and systems, and hardware and/or software for producing and otherwise processing the images or co-operating with the computer program product exemplary embodiments of the application, are not specifically shown or described herein and may be selected from such algorithms, systems, hardware, components and elements known in the art.

Certain exemplary method and/or apparatus embodiments according to the application can provide new dental image acquisition capability in darkroom mode (namely, a minimal GUI interface workspace) and without leaving darkroom mode. Exemplary embodiments according to the application can include various features described herein (individually or in combination). Although embodiments of the present disclosure are illustrated using dental imaging apparatus, similar principles can be applied for other types of diagnostic imaging and for other anatomy.

An exemplary dental image viewing system embodiment can include a source of images, a computer having a processor and an user interface module, the user interface module configured to generate a graphical user interface, the graphical user interface having a first window in which at least one representation or thumbnail from the source images is displayed, and a screen connected to the computer and configured to display the graphical user interface, where, in response to at least one user input, the graphical user interface is configured to generate a second window and a secondary user input action in the second window is configured to launch image acquisitions and display the newly acquired images in an image viewing workspace thereof (e.g., the first window). In one embodiment, an image viewing workspace area in a darkroom mode is dedicated to displaying images one at a time, where each image is preferably displayed at its maximized full-screen size compared to the allocated screen space. Preferably, the graphical user interface does not contain any application frame nor title bar, and thus, can be maximized.

While the invention has been illustrated with respect to one or more implementations, alterations and/or modifications can be made to the illustrated examples without departing from the scope of the appended claims. In addition, while a particular feature of the invention can have been disclosed with respect to only one of several implementations/embodiments, such feature can be combined with one or more other features of the other implementations/embodiments as can be desired and advantageous for any given or particular function. The term "at least one of" is used to mean one or more of the listed items can be selected. The term "about" indicates that the value listed can be somewhat altered, as long as the alteration does not result in nonconformance of the process or structure to the illustrated embodiment. Finally, "exemplary" indicates the description is used as an example, rather than implying that it is an ideal. Other embodiments of the invention will be apparent to those skilled in the art from consideration of the specification and practice of the invention disclosed herein. It is intended that the specification and examples be considered as exemplary only, with a true scope of the invention being indicated by at least the following claims.

## Claims

1. A dental imaging apparatus (18) comprising:
at least one dental image acquisition device (12, 14, 16) that is configured to acquire an image of patient dentition; and
a host processor (20) that is in signal communication with a display (26) and is energizable to generate a graphical user interface (28) on the display, the graphical user interface (GUI) comprising a darkroom mode GUI configured to display one selected diagnostic image of one or more diagnostic images of a selected patient, the darkroom mode GUI comprising:
a display area configured to display the selected diagnostic image among the one or more diagnostic images of the selected patient,
a first user control configured to exit the darkroom mode GUI upon selection,
a second user control configured to alternate the selected displayed diagnostic image in the display area of the darkroom mode GUI among the one or more diagnostic images of the selected patient, and
a third user control configured to prompt an operator of one of the at least one dental image acquisition device to manually energize dental image acquisition by the one of the at least one dental image acquisition device,
where the one of the at least one dental image acquisition device is in signal communication with the darkroom mode GUI, and where the third user control is selectable only when the one of the at least one dental image acquisition device is capable of immediate dental image acquisition of the selected patient.

2. The apparatus of claim 1, where an immediate dental image acquisition 2D or 3D result is to be displayed as the selected displayed diagnostic image among two or more diagnostic images of the selected patient without leaving the darkroom mode GUI.

3. The apparatus of claim 2, where the third user control in the darkroom mode GUI is selectable by the operator of the one of the at least one dental image acquisition device.

4. The apparatus of claim 1, where the darkroom mode GUI is configured to display only one selected diagnostic image of the, one or more diagnostic images of the selected patient.

5. The apparatus of claim 1, where the darkroom mode GUI further comprises an overlay in which at least one thumbnail image of the one or more diagnostic images is displayed.

6. The apparatus of claim 5, where, in use, selection of at least one control button activates a link with the one of the at least one dental image acquisition device and displays a patient image acquired from the one of the at least one dental image acquisition device and displays properties of the one of the at least one dental image acquisition device and of the displayed patient image.

7. The apparatus of claim 1 wherein the selected displayed diagnostic image extends to the dimensional limits of the display.

8. The apparatus of claim 6 wherein the graphical user interface is further configured to display one or more measurement utilities.

9. The apparatus of claim 6 wherein, in response to selection of the at least one control button, the graphical user interface generates a window for acquisition parameter entry.

10. The apparatus of claim 5, where, in use, selection of a thumbnail image activates display of the corresponding image.

11. The apparatus of claim 5 wherein the display is a touch screen display configured to accept operator instructions.

12. The apparatus of claim 5 where, in use, selection of a toolbar menu representation displays the selection of a drawing and measurements toolbar, an image acquisition toolbar, a standard image viewer workspace toolbar, a share toolbar or a patient information toolbar.

13. The apparatus of claim 5, where one or more diagnostic images of the selected patient are selectable in a standard image view and displayable in the darkroom mode GUI.

14. The apparatus of claim 5 comprising:
a first intra-oral image acquisition device that is configured to acquire one or more reflectance images of the patient dentition; and
a second extra-oral image acquisition device that is configured to acquire one or more radiographic images of the patient dentition;
where the host processor is in signal communication with both the first intra-oral image acquisition device and the second extra-oral acquisition device.

15. A method for dental imaging comprising:
providing at least one dental image acquisition device (12, 14, 16) that acquires an image of patient dentition; and
energizing a host processor (20) that is in signal communication with a display (26) and with the at least one dental image acquisition device to generate a graphical user interface (28) on the display, the graphical user interface (GUI) comprising a darkroom mode GUI configured to display one selected diagnostic image of one or more diagnostic images of a selected patient, the darkroom mode GUI comprising:
a display area to display the selected diagnostic image among the one or more diagnostic images of the selected patient,
a first user control configured to exit the darkroom mode GUI upon selection,
a second user control configured to alternate the selected displayed diagnostic image in the display area of the darkroom mode GUI among the one or more diagnostic images of the selected patient, and
a third user control configured to prompt an operator of one of the at least one dental image acquisition device to manually energize dental image acquisition by the one of the at least one dental image acquisition device,
where the one of the at least one dental image acquisition device is in signal communication with the darkroom mode GUI, and where the third user control is selectable only when the one of the at least one dental image acquisition device is capable of immediate dental image acquisition of the selected patient.

## Patentansprüche

1. Dentalbildgebungseinrichtung (18), umfassend:
mindestens eine Dentalbilderfassungsvorrichtung (12, 14, 16), die konfiguriert ist, ein Bild eines Patientengebisses zu erfassen; und
einen Host-Prozessor (20), der sich in Signalkommunikation mit einer Anzeige (26) befindet und energetisierbar ist, um eine grafische Benutzeroberfläche (28) auf der Anzeige zu erzeugen, wobei die grafische Benutzeroberfläche (GUI) eine Dunkelkammermodus-GUI umfasst, die konfiguriert ist, ein ausgewähltes diagnostisches Bild von einem oder mehreren diagnostischen Bildern eines ausgewählten Patienten anzuzeigen, wobei die Dunkelkammermodus-GUI umfasst:
einen Anzeigebereich, der konfiguriert ist, das ausgewählte diagnostische Bild unter dem einen oder den mehreren diagnostischen Bildern des ausgewählten Patienten anzuzeigen,
eine erste Benutzersteuerung, die konfiguriert ist, die Dunkelkammermodus-GUI bei Auswahl zu beenden,
eine zweite Benutzersteuerung, die konfiguriert ist, das ausgewählte angezeigte diagnostische Bild im Anzeigebereich der Dunkelkammermodus-GUI unter dem einen oder den mehreren diagnostischen Bildern des ausgewählten Patienten zu alternieren, und
eine dritte Benutzersteuerung, die konfiguriert ist, einen Bediener von einer der mindestens einen Dentalbilderfassungsvorrichtung aufzufordern, die Dentalbilderfassung durch die eine der mindestens einen Dentalbilderfassungsvorrichtung manuell zu energetisieren,
wobei sich die eine der mindestens einen Dentalbilderfassungsvorrichtung in Signalkommunikation mit der Dunkelkammermodus-GUI befindet, und wobei die dritte Benutzersteuerung nur auswählbar ist, wenn die eine der mindestens einen Dentalbilderfassungsvorrichtung zur sofortigen Dentalbilderfassung des ausgewählten Patienten fähig ist.

2. Einrichtung nach Anspruch 1, wobei ein 2D- oder 3D-Ergebnis der sofortigen Dentalbilderfassung als das ausgewählte angezeigte diagnostische Bild unter zwei oder mehr diagnostischen Bildern des ausgewählten Patienten angezeigt werden soll, ohne die Dunkelkammermodus-GUI zu verlassen.

3. Einrichtung nach Anspruch 2, wobei die dritte Benutzersteuerung in der Dunkelkammermodus-GUI durch den Bediener der einen der mindestens einen Dentalbilderfassungsvorrichtung auswählbar ist.

4. Einrichtung nach Anspruch 1, wobei die Dunkelkammermodus-GUI konfiguriert ist, nur ein ausgewähltes diagnostisches Bild des einen oder der mehreren diagnostischen Bilder des ausgewählten Patienten anzuzeigen.

5. Einrichtung nach Anspruch 1, wobei die Dunkelkammermodus-GUI ferner ein Overlay umfasst, in dem mindestens ein Thumbnail-Bild des einen oder der mehreren diagnostischen Bilder angezeigt wird.

6. Einrichtung nach Anspruch 5, wobei im Gebrauch die Auswahl mindestens einer Steuertaste eine Verknüpfung mit der einen der mindestens einen Dentalbilderfassungsvorrichtung aktiviert und ein Patientenbild anzeigt, das von der einen der mindestens einen Dentalbilderfassungsvorrichtung erfasst wird, und Eigenschaften der einen der mindestens einen Dentalbilderfassungsvorrichtung und des angezeigten Patientenbildes anzeigt.

7. Einrichtung nach Anspruch 1, wobei sich das ausgewählte angezeigte diagnostische Bild zu den Abmessungsgrenzen der Anzeige erstreckt.

8. Einrichtung nach Anspruch 6, wobei die grafische Benutzeroberfläche ferner konfiguriert ist, ein oder mehrere Messdienstprogramme anzuzeigen.

9. Einrichtung nach Anspruch 6, wobei, als Reaktion auf die Auswahl der mindestens einen Steuertaste, die grafische Benutzeroberfläche ein Fenster zur Eingabe von Erfassungsparametern erzeugt.

10. Einrichtung nach Anspruch 5, wobei im Gebrauch die Auswahl eines Thumbnail-Bildes die Anzeige des entsprechenden Bildes aktiviert.

11. Einrichtung nach Anspruch 5, wobei die Anzeige eine Touchscreen-Anzeige ist, die konfiguriert ist, Bedieneranweisungen anzunehmen.

12. Einrichtung nach Anspruch 5, wobei im Gebrauch die Auswahl einer Symbolleistenmenürepräsentation die Auswahl einer Zeichnungs- und Messsymbolleiste, einer Bilderfassungssymbolleiste, einer Standard-Bildbetrachtungsarbeitsbereich-Symbolleiste, einer Sharing-Symbolleiste oder einer Patienteninformationssymbolleiste anzeigt.

13. Einrichtung nach Anspruch 5, wobei ein oder mehrere diagnostische Bilder des ausgewählten Patienten in einer Standard-Bildansicht auswählbar und in der Dunkelkammermodus-GUI anzeigbar sind.

14. Einrichtung nach Anspruch 5, umfassend:
eine erste intraorale Bilderfassungsvorrichtung, die konfiguriert ist, ein oder mehrere Reflexionsbilder des Patientengebisses zu erfassen; und
eine zweite extraorale Bilderfassungsvorrichtung, die konfiguriert ist, ein oder mehrere radiographische Bilder des Patientengebisses zu erfassen;
wobei sich der Host-Prozessor in Signalkommunikation mit sowohl der ersten intraoralen Bilderfassungsvorrichtung als auch der zweiten extraoralen Erfassungsvorrichtung befindet.

15. Verfahren zur Dentalbildgebung, umfassend:
Bereitstellen mindestens einer Dentalbilderfassungsvorrichtung (12, 14, 16), die ein Bild eines Patientengebisses erfasst; und
Energetisieren eines Host-Prozessors (20), der sich in Signalkommunikation mit einer Anzeige (26) und mit der mindestens einen Dentalbilderfassungsvorrichtung befindet, um eine grafische Benutzeroberfläche (28) auf der Anzeige zu erzeugen, wobei die grafische Benutzeroberfläche (GUI) eine Dunkelkammermodus-GUI umfasst, die konfiguriert ist, ein ausgewähltes diagnostisches Bild von einem oder mehreren diagnostischen Bildern eines ausgewählten Patienten anzuzeigen, wobei die Dunkelkammermodus-GUI umfasst:
einen Anzeigebereich zum Anzeigen des ausgewählten diagnostischen Bildes unter dem einen oder den mehreren diagnostischen Bildern des ausgewählten Patienten,
eine erste Benutzersteuerung, die konfiguriert ist, die Dunkelkammermodus-GUI bei Auswahl zu beenden,
eine zweite Benutzersteuerung, die konfiguriert ist, das ausgewählte angezeigte diagnostische Bild im Anzeigebereich der Dunkelkammermodus-GUI unter dem einen oder den mehreren diagnostischen Bildern des ausgewählten Patienten zu alternieren, und
eine dritte Benutzersteuerung, die konfiguriert ist, einen Bediener von einer der mindestens einen Dentalbilderfassungsvorrichtung aufzufordern, die Dentalbilderfassung durch die eine der mindestens einen Dentalbilderfassungsvorrichtung manuell zu energetisieren,
wobei sich die eine der mindestens einen Dentalbilderfassungsvorrichtung in Signalkommunikation mit der Dunkelkammermodus-GUI befindet, und wobei die dritte Benutzersteuerung nur auswählbar ist, wenn die eine der mindestens einen Dentalbilderfassungsvorrichtung zur sofortigen Dentalbilderfassung des ausgewählten Patienten fähig ist.

## Revendications

1. Appareil d'imagerie dentaire (18) comprenant :
au moins un dispositif d'acquisition d'image dentaire (12, 14, 16) qui est configuré pour acquérir une image de la dentition d'un patient ; et
un processeur hôte (20) qui est en communication de signal avec un afficheur (26) et est apte à être amorcé pour générer une interface utilisateur graphique (28) sur l'afficheur, l'interface utilisateur graphique (GUI) comprenant une GUI en mode chambre noire configurée pour afficher une image de diagnostic sélectionnée d'une ou de plusieurs images de diagnostic d'un patient sélectionné, la GUI en mode chambre noire comprenant :
une zone d'affichage configurée pour afficher l'image de diagnostic sélectionnée parmi les une ou plusieurs images de diagnostic du patient sélectionné,
une première commande d'utilisateur configurée pour sortir de la GUI en mode chambre noire lors d'une sélection,
une deuxième commande d'utilisateur configurée pour alterner l'image de diagnostic affichée sélectionnée dans la zone d'affichage de la GUI en mode chambre noire parmi les une ou plusieurs images de diagnostic du patient sélectionné, et
une troisième commande d'utilisateur configurée pour inviter un opérateur, d'un dispositif de l'au moins un dispositif d'acquisition d'image dentaire, à amorcer manuellement une acquisition d'image dentaire, par le dispositif de l'au moins un dispositif d'acquisition d'image dentaire,
où le dispositif de l'au moins un dispositif d'acquisition d'image dentaire est en communication de signal avec la GUI en mode chambre noire, et où la troisième commande d'utilisateur est apte à être sélectionnée uniquement lorsque le dispositif de l'au moins un dispositif d'acquisition d'image dentaire est capable d'une acquisition d'image dentaire immédiate du patient sélectionné.

2. Appareil selon la revendication 1, où un résultat en 2D ou 3D d'acquisition d'image dentaire immédiate doit être affiché comme étant l'image de diagnostic affichée sélectionnée parmi deux images de diagnostic ou plus du patient sélectionné sans quitter la GUI en mode chambre noire.

3. Appareil selon la revendication 2, où la troisième commande d'utilisateur dans la GUI en mode chambre noire est apte à être sélectionnée par l'opérateur du dispositif de l'au moins un dispositif d'acquisition d'image dentaire.

4. Appareil selon la revendication 1, où la GUI en mode chambre noire est configurée pour afficher une seule image de diagnostic sélectionnée des une ou plusieurs images de diagnostic du patient sélectionné.

5. Appareil selon la revendication 1, où la GUI en mode chambre noire comprend en outre une incrustation dans laquelle au moins une vignette des une ou plusieurs images de diagnostic est affichée.

6. Appareil selon la revendication 5, où, en utilisation, la sélection d'au moins un bouton de commande active un lien avec le dispositif de l'au moins un dispositif d'acquisition d'image dentaire et affiche une image de patient acquise à partir du dispositif de l'au moins un dispositif d'acquisition d'image dentaire et affiche des propriétés du dispositif de l'au moins dispositif d'acquisition d'image dentaire et de l'image de patient affichée.

7. Appareil selon la revendication 1 dans lequel l'image de diagnostic affichée sélectionnée s'étend jusqu'aux limites dimensionnelles de l'afficheur.

8. Appareil selon la revendication 6 dans lequel l'interface utilisateur graphique est en outre configurée pour afficher un ou plusieurs utilitaires de mesure.

9. Appareil selon la revendication 6 dans lequel, en réponse à la sélection de l'au moins un bouton de commande, l'interface utilisateur graphique génère une fenêtre pour la saisie d'un paramètre d'acquisition.

10. Appareil selon la revendication 5, où, en utilisation, la sélection d'une vignette active un affichage de l'image correspondante.

11. Appareil selon la revendication 5 dans lequel l'afficheur est un afficheur à écran tactile configuré pour accepter des instructions d'opérateur.

12. Appareil selon la revendication 5 où, en utilisation, la sélection d'une représentation de menu de barre d'outils affiche la sélection d'une barre d'outils de dessin et de mesures, d'une barre d'outils d'acquisition d'image, d'une barre d'outils d'espace de travail de visionneuse d'images standard, d'une barre d'outils de partage ou d'une barre d'outils d'informations sur le patient.

13. Appareil selon la revendication 5, où une ou plusieurs images de diagnostic du patient sélectionné sont aptes à être sélectionnées dans une vue d'image standard et à être affichées dans la GUI en mode chambre noire.

14. Appareil selon la revendication 5 comprenant :
un premier dispositif intra-oral d'acquisition d'images qui est configuré pour acquérir une ou plusieurs images par réflectance de la dentition du patient ; et
un second dispositif extra-oral d'acquisition d'images qui est configuré pour acquérir une ou plusieurs images radiographiques de la dentition du patient ;
où le processeur hôte est en communication de signal avec à la fois le premier dispositif intra-oral d'acquisition d'images et le second dispositif extra-oral d'acquisition.

15. Procédé d'imagerie dentaire comprenant :
la fourniture d'au moins un dispositif d'acquisition d'image dentaire (12, 14, 16) qui acquiert une image de la dentition d'un patient ; et
l'amorçage d'un processeur hôte (20) qui est en communication de signal avec un afficheur (26) et avec l'au moins un dispositif d'acquisition d'image dentaire pour générer une interface utilisateur graphique (28) sur l'afficheur, l'interface utilisateur graphique (GUI) comprenant une GUI en mode chambre noire configurée pour afficher une image de diagnostic sélectionnée d'une ou de plusieurs images de diagnostic d'un patient sélectionné, la GUI en mode chambre noire comprenant :
une zone d'affichage pour afficher l'image de diagnostic sélectionnée parmi les une ou plusieurs images de diagnostic du patient sélectionné,
une première commande d'utilisateur configurée pour sortir de la GUI en mode chambre noire lors d'une sélection,
une deuxième commande d'utilisateur configurée pour alterner l'image de diagnostic affichée sélectionnée dans la zone d'affichage de la GUI en mode chambre noire parmi les une ou plusieurs images de diagnostic du patient sélectionné, et
une troisième commande d'utilisateur configurée pour inviter un opérateur, d'un dispositif de l'au moins un dispositif d'acquisition d'image dentaire, à amorcer manuellement une acquisition d'image dentaire, par le dispositif de l'au moins un dispositif d'acquisition d'image dentaire,
où le dispositif de l'au moins un dispositif d'acquisition d'image dentaire est en communication de signal avec la GUI en mode chambre noire, et où la troisième commande d'utilisateur est apte à être sélectionnée uniquement lorsque le dispositif de l'au moins un dispositif d'acquisition d'image dentaire est capable d'une acquisition d'image dentaire immédiate du patient sélectionné.
